# EUROPEAN PATENT SPECIFICATION

(11) **EP 4 619 085 B1**
(45) Date of publication and mention of the grant of the patent: **15.07.2026**
(21) Application number: 23806390.3
(22) Date of filing: 14.11.2023
(51) Int. Cl.: A61N 1/05, A61B 5/251, A61B 5/388, A61B 5/00

(54) **SELF-INSERTING PERIPHERAL NEURAL INTERFACE**
SELBSTEINSETZENDE PERIPHERE NEURONALE SCHNITTSTELLE
INTERFACE NEURONALE PÉRIPHÉRIQUE À AUTO-INSERTION

(30) Priority: 15.11.2022 IT 202200023493
(43) Date of publication of application: 24.09.2025
(73) Proprietor: Scuola Superiore di Studi Universitari e di Perfezionamento Sant'Anna, 56127 Pisa (IT); INAIL - Istituto Nazionale per l'Assicurazione contro gli Infortuni sul Lavoro, 00187 Roma (RM) (IT); Fondazione Istituto Italiano di Tecnologia, 16163 Genova (IT)
(72) Inventor: GIANNOTTI, Alice, 56123 Pisa (IT); STRAUSS, Ivo, 56124 Pisa (IT); SINIBALDI, Edoardo, 16163 Genova (IT); MICERA, Silvetro, 50132 Firenze (IT)
(74) Representative: Pietri, Simona
(86) International application number: PCT/IB2023/061481
(87) International publication number: WO 2024/105559

(56) References cited:
- WO-A1-95/10227
- DE-A1- 10 028 522
- US-A1- 2021 252 282

## Description

### Technical field

The present invention relates to a self-inserting peripheral neural interface or electrode capable of an autonomous implanting on a nerve to perform an intraneural-type arrangement.

### Background

Peripheral nervous system is responsible for the connection between brain and organs. Efferent neural signals allow to modulate organs functionality while afferent neural signals allow the brain to monitor organs conditions.

Nerves are mainly composed of several groups of fibers bundled in fascicles, each of them responsible for the control of a specific anatomical structure or function. Three layers of connective tissue support allow the organization of nerves in fascicles. The endoneurium surround each fascicle, then the fascicles are immersed in the perineurium, and the epineurium is wrapped all around this structure, as a protective layer.

Traditionally, peripheral neural interfaces have been developed to deal with situation in which the neural connection is missing due to several pathologies (e.g., traumas, degenerative pathologies). The role of neural interfaces is to record neural signals arousing from peripheral organs using active sites to predict organ state and/or modulate organ functionality by injecting an electrical current within the target nerve.

A wide range of known interfaces has been developed to deal with peripheral nerves anatomy. The classification of them is based on the ratio between invasiveness of the neural interface and their selectivity in nervous fiber recruitment. The more invasive is the device, the more it is able to selectively injects current or record signals from a specific fiber; however, invasiveness also means higher probability to induce trauma to the nerve [1].

Traditionally peripheral nerves interfaces can be grouped in: epineural, extraneural, interfascicular, intrafascicular and regenerative, based on their degree of invasiveness:
- Epineural: this type of interface is placed beside the nerve. One of the larger used in clinical practice is the "tined lead" type; this nerve interface is easy to implant but has a very low selectivity.
- Extraneural: this type of interface surrounds the nerve. The most used in clinical practice is the "cuff" type. The first is in the form of a cylinder wrapped around the nerve [1]. The "cuff" type is easy to implant and can be made with a wide range of materials. The electrical contacts are placed within its internal surface both in radial and longitudinal direction (enabling both targeting different regions of the nerve cross-section both the recording of nerve signals traveling along the nerve axis). Long-term implantation is well-tolerated thanks to the low degree of nerve invasiveness. However, the "cuff" type has low selectivity causing the stimulation of the whole nerve section and/or the recording of Compound Action Potentials only resulting in a low signal-to-noise ratio. Another example of extraneural interface is the "Fine" type which has a higher selectivity compared to the cuff maintaining the easiness of implantation. The "Fine" type as a cuff geometry reshaping the nerve to move the fascicles closer to the electrode contacts However, the higher selectivity is based on nerve compression which induces fascicular position reshaping thus it still is not enough selective and can cause nerve damage.
- Interfascicular: this type of interface can reshape epineurium and nerve geometry displacing nerve fascicles and allowing electrical contacts to increase their proximity with nerve internal fascicles. The known "Spine" type of interfascicular interface is in form of a cuff with extruding contacts. These interfaces have higher selectivity compared to the extraneural type. However, they present some drawbacks; in particular, the contact number is still low, they are difficult to miniaturization. Moreover, they have a long time of implantation.
- Intrafascicular: this intraneural interface also penetrates the epineurium allowing contact nerve internal fascicles. There are several known types of intraneural interfaces as "Life", "Time", "Seline", "Utah array", "Usea array". "Life" is a neural interface with a low number of active sites resulting in average selectivity, already tested long-term in humans [4], [5]. "Time" has an average number of active sites and high neural selectivity tested long-term in humans [4], [6]. Both "Life" and "Time" are biocompatible [7]. "Time" interfaces present the problem that the transverse positioning of electrodes with respect to nerve longitudinal axis is not optimal for either recording nor stimulation in bipolar or tripolar configuration [1]. Furthermore, nerve trauma could be occurred during insertion. "Utah array" and "Usea array" types have a high number of active sites resulting in a high selectivity. They have been tested in humans [8]. However, they are characterised by a high degree of invasiveness and stiffness [9] and they need for a pneumatic inserter to be implanted.
- Regenerative: this type of interface restores the connection between nerve stumps. Some types of regenerative interface are "Sieve", "Microchannel" and "Teeni" [10], [11], [12]. They have a high number of active sites and are highly selective; but they take very long time since nerve has to be regenerated, for the implantation the nerve has to be cut thus they are only suitable for traumatic nerve damage.

Some other examples of known prior art are described below.

In WO2009012502 an implantable electrode is described, comprising a stem and barb structure. The active sites are located on the barb structure which may be less than the stem (<25µm) and is therefore potentially smaller/miniaturized. The device has a morphing behaviour and in particular it changes between two states with respect to the temperature when implanted. Once the barb structure extends, mechanical stability is given. The active sites penetrate the fascicles. This electrode is inserted mechanically, and the active sites are distributed longitudinally but not laterally and therefore not in a 3D manner. The described device is not able to autonomously penetrate the nerve because the morphing mechanism only obtains the electrical connection of the barbs of the electrode but not the implantation inside the nerve. The implantation procedure is not "surgeon-free".

The international publication WO2016005400A1 describes a device for detecting impulses within a nerve. The device comprises a support for securing to the epineurium of the nerve, at least one spike extending from said support, arranged to penetrate the perineurium (intraneural) and at least one chemical sensor arranged on a surface of the at least one spike. The device also comprises three-dimensional (3D) distributed and miniaturized active sites. However, the device has to be inserted manually. There are no functional substances embedded in the electrode reducing neural damage.

Another example of known electrode is described in WO9510227. During the process of closure of the interface around the nerve, the active sites are self-curling to drive into the nerve, however the active sites are not penetrating the perineurium, fascicles are mentioned to be displaced rather than pierced (such as is supposed to happen in intrafascicular interfaces); in addition of that the time of implantation is quite long.

CN104548345A describes another example of implantable electrode with morphing properties. In particular the publication discloses an elastic self-curling coating nerve-implantable connector device. The elastic self-curling coating nerve-implantable connector device comprises a stimulating electrode array unit. Said stimulating electrode array element is the array arrangement that m row n arranges.

US2021/252282 discloses an implantable peripheral neural interface.

### Summary of the invention

The invention is defined by the independent claim 1.

In this context, the addressed technical problem is to overcome one or more of the drawbacks previously mentioned with reference to the known prior art. In particular the technical problem posed and solved by the present invention is to provide a peripheral neural interface that autonomously achieve a selective, stable, and simultaneously safe interaction with nervous fibers when implanting the interface for the long-term application with high-repeatability; wherein "autonomously" means: A) without the need of additional insertion devices, which can cause significant damage or trauma to the nerve and, and B) free from surgeon sensitivity, which can significantly reduce repeatability of the implant procedure.

The use of additional insertion devices or the need for surgeon dexterity may also possibly weaken its market potential on the longer period as well because of cost-effectiveness.

Such a problem is solved by a peripheral neural interface according to claim 1. Preferred features of the present invention are the object of the dependent claims.

### Brief description of the drawings

The present invention and the following detailed description of preferred embodiments thereof may be understood by reference to the following figures:
- Figure 1 shows the matrix 1 of the interface in an open configuration with respect to a nerve N;
- Figure 2 shows external layer and internal layer of the neural interface;
- Figures 3a and 3b show intraneural means of the electrode in a first embodiment;
- Figure 4 shows extraneural means of the neural interface;
- Figures 5a and 5b shows a second embodiment of the intraneural means of the interface with a non-penetrating portion;
- Figures 6a and 6b 3d show a third embodiment of the intraneural means;
- Figure 7 shows a first embodiment of anchoring means of the interface to the nerve N;

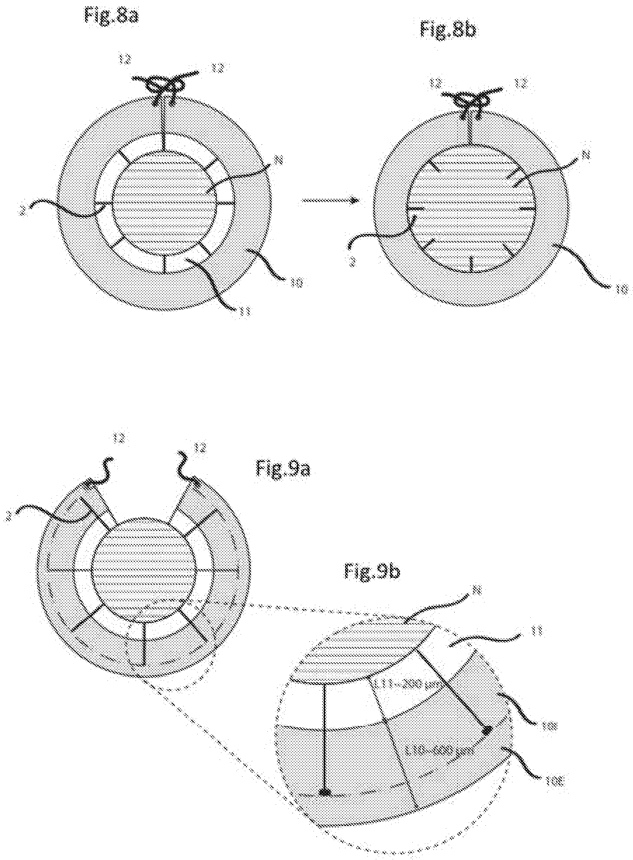

- Figures 8a and 8b show the electrode and in particular the anchoring means and the intraneural means at the initial time of implantation and at the end of the implantation process into the nerve;
- Figures 9a and 9b show another embodiment of the neural interface;
- Figure 10 shows a second embodiment of anchoring means of the interface to the nerve N;
- Figures 11 and 12 show other embodiments of anchoring means of the interface to the nerve N;
- Figures 13, 14 and 15 show different geometry of a penetration portion with linear high aspect ratio of the intraneural means;
- Figures 16a and 16b show another geometry of the penetration portion of the intraneural means made of smart materials.

### Detailed description of preferred embodiments

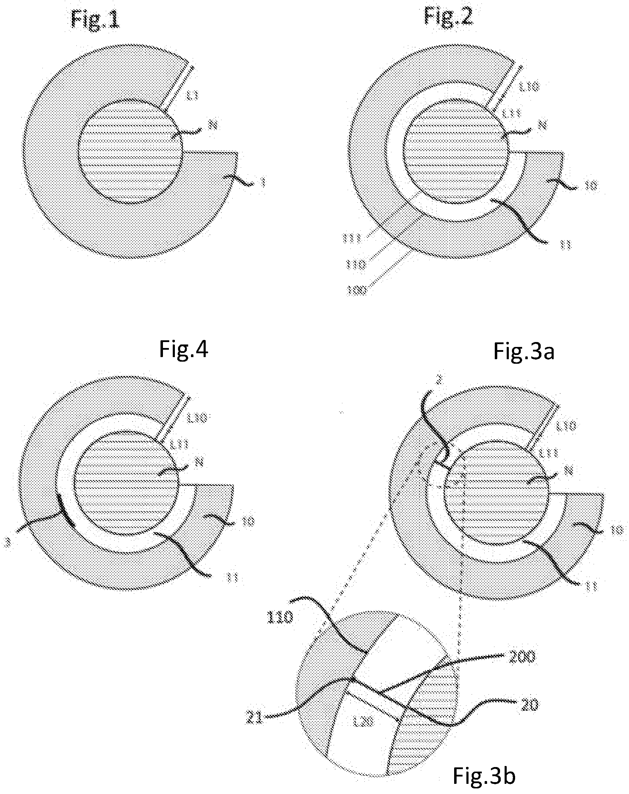

The peripheral neural interface according to the invention comprises a matrix 1 with an own thickness L1 (figure 1). The matrix 1 comprises two overlapping layers each of them made of different materials. In particular, an external layer 10, with a thickness L10, that defines an outer surface 100 of the electrode itself and an internal layer 11, with a thickness L11; wherein L1=L10+L11.

The meaning of the terms "outer" and "inner" is intended in relationship with the arrangement of the interface on the nerve N; in particular, when the interface is implanted the internal layer is in proximity of the nerve surface, instead the outer layer is more distant from the nerve surface.

The internal layer defines a inner surface 111 adapted to contact with the nerve surface.

The internal layer also defines an interfacial surface 110 adapted to contact with the external layer 10.

The matrix 1 and the external layer 10 as consequence, has any geometrical shape which has a nominal diameter comparable with the target nerve diameter; examples of geometrical shapes are cylinder, a cylinder with a lattice structure, a helix, a rectangular structure.

The external layer 10 mechanically anchors the neural interface to the nerve due to its geometrical shape.

The external layer 10 has elastic properties and insulating properties. Suitable materials are, for example, polydimethylsiloxane (PDMS), silicone, etc.

In a further configuration the external layer 10 comprises two or more layers made of the same or different materials. For example, suitable materials are polydimethylsiloxane (PDMS), silicone, etc..

In general, it is preferable that in case of a plurality of layers the external one will be made of a material with at least insulating properties.

With reference to the internal layer 11, according to an aspect of the invention it is made of a degradable biocompatible material. The degradable layer will be also called "sacrificial layer".

In an embodiment of the invention, the degradable material is a biocompatible biodegradable polymer.

A non-limiting example of suitable material is Polyethylene glycol (PEG) which is a flexible, water-soluble, and biocompatible polymer already used for neural applications, as a stiffener [13]-[15].

Other possible solutions are based on the use of silk or chitosan [15], [16].

Another suitable material is for example Polyglicerol sebacate.

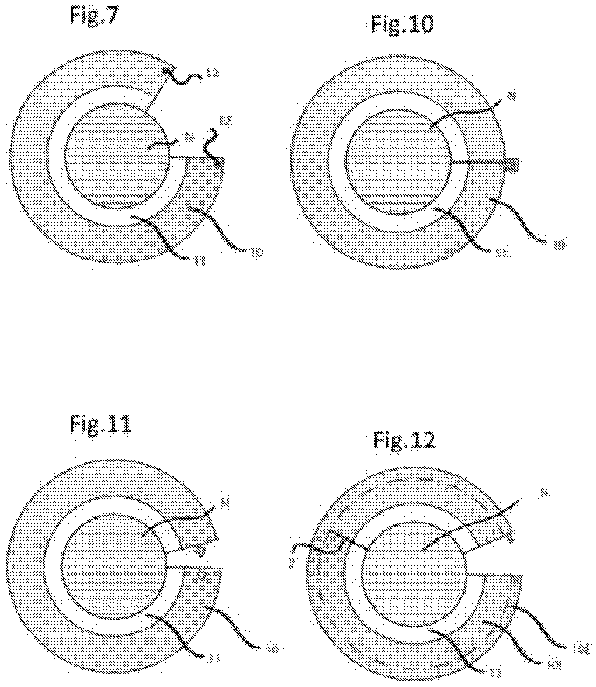

The external layer 10 can have two layer (10I and 10E) and the external one (10I) has to be made with a material with insulating properties (figure 12).

The neural interface also comprises intraneural means 2 to detect a neural signal from nerve and/or inject electrical current, by penetrating the nerve epineurium (figure 3a).

In an preferred embodiment of the invention, intraneural means 2 are constituted of electrically conductive means.

Intraneural means 2 are embedded into the matrix 1.

The intraneural means 2 define a penetration tip 20. When the neural interface is not implanted, the intraneural means 2 is embedded into the internal layer 11. When the interface is implanted into the nerve N, the penetration tip 20 and the intraneural means 2 penetrates the nerve epineurium (figure 3b).

The intraneural means 2 also comprise a fixed tip 21 opposite to the penetration tip 20. The fixed tip 21 is stable connected with the matrix 1.

The fixed tip allows the connection of the intraneural means 2 to an external recording/stimulation device (Figure 3b, 5b and 6b).

In a first configuration of the invention shown in figures 3a and 3b the intraneural means 2 are embedded into the internal layer 11. In this case the fixed tip 21 is stable connected to the interfacial surface 110.

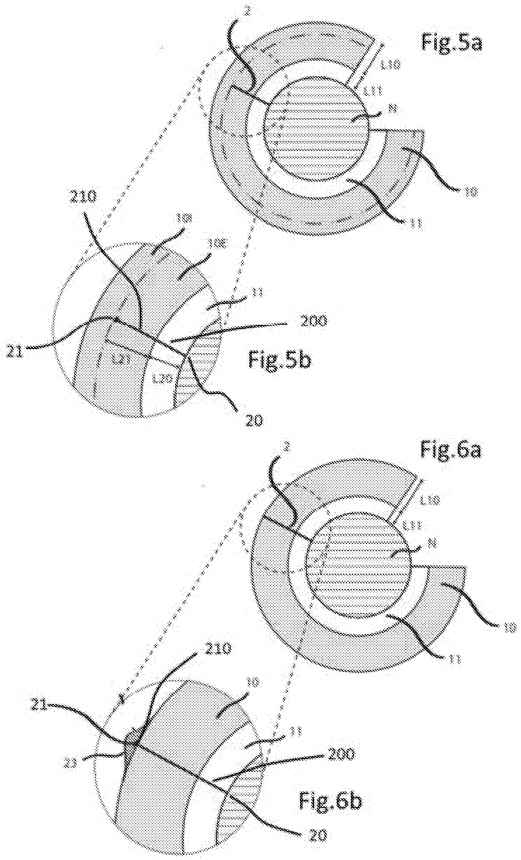

In a further embodiment, the intraneural means 2 defines two portions: a penetration portion 200 comprising the penetration tip 20 and a non-penetration portion 210 comprising the fixed tip 21. The penetration portion 200 penetrates the nerve, instead the non-penetration portion 210 remains out of the nerve during implantation (figure 5a and 5b).

In this embodiment the penetration portion 200 is embedded into the internal layer and the non-penetration portion is embedded in the external layer.

With reference to figures 5a and 5b, in a first configuration of the invention, the non-penetration portion 21 is completely embedded into the external layer 10.

In a further configuration, shown in figures 6a and 6b the non-penetration portion 210 is embedded into the external layer 10 and the fixed tip 21 project out of the external layer on the surface of it. In this case a insulating cover 23 can be provided onto the free end to assure the insulating of it. The insulating cover 23 can be placed locally in correspondence of the fixed tip 21.

Intraneural means 2 comprise for example one or more micrometric high-aspect-ratio conductive linear elements. For example, they are made of micrometric diameter and biocompatible wires. As an example, carbon fibers have already been used to penetrate peripheral nerve thanks to its high elastic modulus (230-250 GPa) [18]-[20].

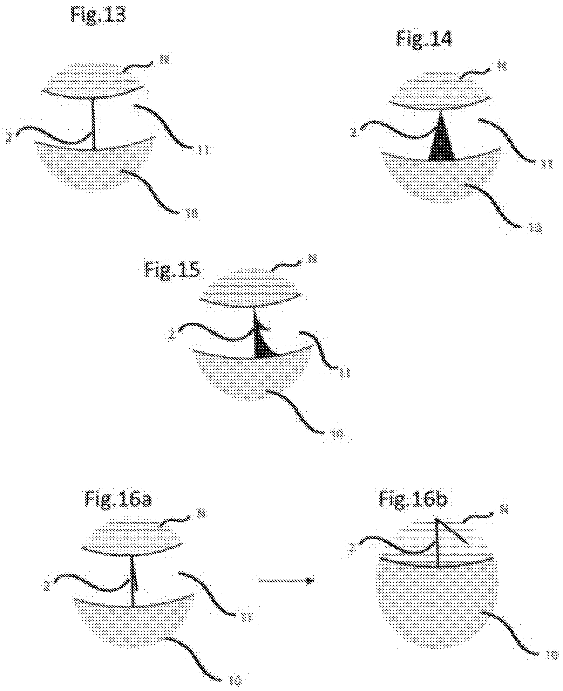

In this example, shown in the figures 13 and 14, the penetration portion has length L20 and the non-penetration portion, where presents, has length L21.

In general, the height of intraneural means is such that when the nerve is penetrated, the conductive portions does not overcome the nerve radius.

In another embodiment of the invention, intraneural means are made using additive manufacturing techniques with nanometric resolution, such as two-photons lithography or three-dimensional photolithography (e.g. nanoscribe or 3D printing) [21].

For example, they can be made with epoxy-based negative photoresist (such as SU-8) covered with metallic layer (such as gold), (Figures 14, 15) in order to obtain a hooked shape adapted to avoid the undesired extraction of the penetration portion.

Other examples of suitable materials for the making of the intraneural means are smart materials, for example conductive materials like shape memory alloys, capable to change shape in function of a temperature variation.

Examples of intraneural means 2 obtained with smart materials are shown in figures 16a and 16b where it is visible the opening movement of the penetration portion when inside to the nerve, in order to obtain a hooked shape and therefore avoiding the undesired extraction of the conductive element itself.

Each conductive element projects from the inner surface 111 of the internal layer 11 in a perpendicular direction when the layer is in a planar arrangement, therefore in a radial arrangement when the neural interface is disposed around the nerve.

The intraneural means 2 can be positioned and distributed in a custom configuration on the nerve cross-section in order to achieve the highest possible neural selectivity. The intraneural means, when the device is implanted, are distributed within the nerve cross-section such that the largest part of the nerve area is covered.

Again, the intraneural means 2 can be positioned in a custom configuration on the longitudinal nerve section. When the device is implanted, they have more than one active site distributed within the nerve axis to allow also bipolar or tripolar recording.

Anchoring or suturing means 12 for the stable fixing of the neural interface to the nerve are associated to the external layer 10.

In an embodiment anchoring means comprises suturing wires 12 projecting by the opposite end of the external layer (figure 7).

Other possible examples for the means fixing the external layer 10 to the nerve can involve the use of a hook structure (figure 10), a clip structure (figures 11, 12), or a magnetic mechanism.

According to another configuration of the neural interface, it also comprises detecting/stimulating extraneural means 3 to detect a neural signal from nerve and/or inject electrical current, by surrounding the nerve epineurium. The detecting/stimulating extraneural means 3 are displaced on the surface 110 (Figure 4).

In another embodiment of the invention, optical fibers for optogenetic stimulation or microfluidic delivery are provided with intraneural means 2.

During the implantation procedure on the nerve N, the neural interface is wrapped around the nerve N by gently pulling its extremities and slightly increasing its nominal internal diameter thanks to the elasticity of the material constituting the external layer 10. The implanted external layer wrapped around the nerve N is deformed from its nominal diameter in the range of small deformations.

When the sacrificial layer 11 comes in contact with the neural tissue, it gradually degrades, causing the reducing of the sacrificial layer thickness L11; consequently, the penetration portion 20 of the intraneural means 2 gradually is exposed out of the sacrificial layer 11 and therefore it penetrates the nerve.

The progressive exposure of the intraneural elements allows the self-penetration of them within the nerve fascicles. While the exposed penetration portion of each intraneural element pierces the neural tissue, the residual sacrificial layer acts as a supporting material avoiding bending and/or fracture of the conductive elements.

During the degradation of the sacrificial layer 11 the external layer 10 wrapped around the nerve applies a compression onto the nerve itself. The compression results as a consequence of its deformed state from its nominal diameter, due to the elasticity properties of the material of which the external layer is made. The compression force exerted by the external layer 10 pushes the conductive elements 2 through the neural tissue of the nerve.

At the same time, nervous tissue swells as a consequences of the trauma induced from through the implantation of the device. The swelling process works in the opposite direction of compression, helping the penetration process. The natural swelling process allows to reduce the compression force required to insert the intraneural means 2 within the nerve N.

Once the intraneural elements have penetrated the nerve with their penetration portion 20, neural signals from nerve fibers and/or can be recorded or electrical current can be injected into the nerve. At this time, the described neural interface can be considered to be an intraneural-type neural interface.

Rewording, the mechanism of action consists of the following steps:
a. The neural interface is wrapped around the nerve exerting an elastic enlargement of the external layer; as a consequence, the interface exerts compression onto the nerve. In the meanwhile, the implanted nerve undergoes a swelling process as a physiological response to the contact with the interface.
b. The sacrificial layer 11 undergoes degradation as soon as in contact with the physiological environment. Therefore, the autonomous morphing process starts. Consequently, penetration portions 20 of conductive elements 2 embedded within the internal layer 11 are exposed and come in contact with the nervous tissue.
c. The interface is implanted through the combination of the two opposite direction forces described at step 1 (compressive force on the nerve and swelling process of the nerve) which allow the progressive self-penetration of the conductive penetration portions 20 of the intraneural means 2. The gradual degradation of the sacrificial layer 11 prevents the conductive elements bending and/or fracture.

### Example of an embodiment of the invention:

The following example shown in Figure 7 describes a possible configuration, of the described device according to the invention.

Considering a small nerve having a 500 µm mean radius, the height of the portion 20 of the intraneural means which penetrate the nerve could be realized in the order of 200 µm. Thus, the starting sacrificial layer height L11 is at least 200 µm to completely embeds the intraneural means 2. By choosing carbon fibers as the material to realize the intraneural elements 2, the insulating portion 10 has a height equal to at least 2/3 of the penetrating portion of the conductive elements in order to avoid carbon fibers fracture (tensile strength 4.5 GPa), then L10 is at least 600 µm. This value is comparable with extraneural cuff interface commercially available. Then, the total intraneural means 2 length is at least 800 µm.

### Possible fabrication methods:

Method 1: Fabrication of the interface by using an array of single carbon fibers as active sites integrated within a PDMS substrate casted with photolithographic techniques.

The process provides the use of traditional photolithographic techniques to realize a PDMS structure (external layer 10) with micrometrics holes (order of 10 µm) housing carbon fibers (electrical penetrating conductive means 2). As for all the traditional photolithographic techniques the PDMS patterning are performed on a silicon wafer.

Briefly, the fabrication process comprises the step of:
1) Pre-treatment of the silicon wafer (dehydration) at 120 C° for 120 seconds. 2) Spin coating of 150 µm of negative photoresist (e.g., SU-8 2050) on the silicon wafer (e.g., spin speed 1250 rpm for 8 seconds). 3) UV-exposure of photoresist for 10 seconds, using a mask-aligner in soft contact mode and a photolithographic mask to realize an array of holes (10 µm diameter). 4) Dry O₂ etching of the exposed surface to remove SU-8 and open round structures. 5) Insertion of 200 µm long, 8 µm thick carbon fibers into the 10 µm openings. Fixation of carbon fibers within the holes with biocompatible biodegradable material (e.g., polyethylene glycol 3350 (PEG)) to hold the in position. The electrically conductive means 2 are made of carbon fibers. 6) In order to obtain the external layer 10, spin coating of Polydimethylsiloxane (PDMS) at 4200 rpm for 10 seconds. Baking at 120C° for 15 minutes. 7) Deposition of PDMS-platinum (100 nm thickness) and sputtering of activated iridium oxide film (250 nm) to realize electrical connection of the device with the external stimulator. 8) Repetition of step 6 to cover the device with PDMS and electrically insulate the device. 9) Lift-off of SU-8 layer and detachment of the device from the silicon wafer. 10) Spin coating of biocompatible biodegradable elastomeric material (e.g., polyglycol sebacate) at 1200 rpm for 10 seconds on the fibers to realize the internal sacrificial layer 11. 11) Interfacing with device through manually soldering 120 diameter copper micro-wires to the realized electrical connection.

Method 2: Fabrication of SpiCE interface by using an array of carbon-fibers bundles as active sites integrated within a PDMS substrate casted with additive manufacturing techniques.

The process comprises the use of additive manufacturing techniques (e.g., stereolithography) to realize a resin mold for the casting of a PDMS structure (external layer 10) with micrometrics holes (order of 100 µm) housing bundle of carbon fibers (electrical penetrating conductive means 2).

Briefly, the fabrication process comprises the following steps:
1) Dip coating of around 10 carbon fibers into polyethylene glycol 3350 (PEG) to realize a 100 µm diameter carbon-fiber bundle and curing at room temperature for 120 minutes. Repetition of previous step to have multiple carbon-fiber bundles. Carbon-fiber bundles materialize the electrically conductive means 2. 2) Cutting carbon-fiber bundles into a 200 µm long pieces. 3) Realization with stereolithography of a resin mold made of two pieces. Holes of 100/150 µm in diameter are constructed within the mold to insert metallic needles. 4) Insertion of one carbon-fiber bundle into each needle. 5) Injection of PDMS into the mold and polymerization at 120 C° for 60 minutes. The PDMS structure is the external layer 10. 6) Cooling down the mold to room temperature and extraction of PDMS structure with carbon-fiber bundles embedded. 7) Extraction of needles allowing carbon-fiber bundle to stay within the PDMS. 8) Integration of the system containing the carbon-fiber bundle into a cuff system. 9) Spin coating of biocompatible biodegradable elastomeric material (e.g., polyglycol sebacate) at 1200 rpm for 10 seconds on the fibers to obtain the internal sacrificial layer 11.

Method 3: Fabrication of the interface by using an array of high-aspect ratio intraneural conductive means 2 as active sites realized using two-photon lithographic techniques integrated within a polyimide substrate casted with traditional photolithographic techniques.

The process comprises the use of traditional photolithographic techniques to realize a polyimide structure (external layer 10) with bidimensional electrical contacts. Parts of the conductive means will constitute electrically conductive means 3. On the top of the remaining three-dimensional 3D penetrating electrically conductive means will be realized by using high-resolution two-photon lithographic techniques.

Briefly, the fabrication process comprises the following steps:
1) A thin-film, multi-site electrode based on a polyimide substrate is realized using thin-film microfabrication techniques. The polyimide substrate is the external layer 10. Parts of the contacts within the realized multi-site electrode is electrically conductive means 3. 2) Multi-site electrode is mounted on a thin optical glass and a drop of liquid acrylic photoresist (e.g., IP-Dip, Nanoscribe, GmbH) is deposited over the multi-site electrode, 3) The 3D penetrating elements (that will constitute means 2) are printed through the optical glass substrate using a two-photon-polymerization-based, dip-in resonant direct laser writing (rDWL) process (e.g., Nanoscribe, GmbH). 4) Printed samples are developed (e.g., using propylene glycol monomethyl ether acetate, PGMEA in a 25 mL beaker for 10 minutes). 5) Deposition of 100 nm platinum using evaporation on top of the 3D penetrating elements, and consequent deposition of Iridium Oxide (Ir-Ox) through electrochemical deposition in a standard three-electrode setup. 3D printed penetrating elements coated with Ir-Ox are the electrically conductive means 2. 4) Spin coating of biocompatible biodegradable elastomeric material (e.g., polyglycol sebacate) at 1200 rpm for 10 seconds on the fibers to realize the internal sacrificial layer 11.

The described device obtains several advantages.

The sacrificial layer allows an innovative non-claimed method for the easy implantation of intraneural interfaces within small nerves in a small operative surgical area for long-term application, reducing the total tissue exposure during the surgery and therefore reducing tissue damage.

The sacrificial layer also avoids the sensitivity to surgical operator skills thus reducing the outcome variability. The repeatability introduced by the sacrificial layer enable the present invention to be industrially competitive.

The sacrificial layer 11 achieves two main goals: 1) it protects the intraneural means 2 from bending during implantation allowing a fast and simple implantation procedure; 2) it provides assistance to the intraneural means 2 allowing their self-penetration.

The mechanical action underlying the morphing procedure, i.e., the sacrificial layer degradation, is thought to be passive in order to achieve the higher order of simplicity. The integration of other morphing strategies does not affect the core aspects of the proposed technical solution.

The sacrificial layer dissolution time defines the characteristic implantation time. allowing the fast insertion (in the order of minutes) of one or multiple intraneural means, reducing tissue exposure and, thus, possible infections.

The degradation time of the sacrificial layer 11 is in the order of minutes. Therefore, mechanical anchoring of the electrode without bending of intraneural means can be assured during implantation. Contemporary, this is a period short enough to avoid too long surgeries and nerve compression.

Moreover, the sacrificial layer allows the nerve penetration avoiding the need for extra assistant tools (such as inserters and/or micromanipulators) and reducing surgical manipulation of the nerve, finally reducing the chance of nerve trauma.

Advantageously, the sacrificial layer can integrate substances, such as collagenase, which can reduce the epineurium stiffness and thus reducing the penetration process complexity [17].

The sacrificial layer 11 or the intraneural means 2 can embed or deliver drugs for nerve repairing, reducing post-surgery trauma.

The present neural interface configuration and non-claimed implantation method is a scalable to every nerve diameter, by customizing each elements dimension. This to safely apply an intraneural approach also to small nerves, which otherwise are easy to damage and difficult to implant. Furthermore, the autonomous implantation of penetrating portion reduces the need for surgeon, avoiding the repeatability of the process to be affected by surgeon.

The combination of an external layer 10 to anchor the nerve, a intraneural means 2, which penetrate it allows the stability of the interface in long-term implant, avoiding interface dislocation from implant site and reducing nerve trauma and decrease fibrotic tissue development.

The external layer 10 and suturing or anchoring means 12 provides mechanical stability to the implant avoiding unwanted movements of the intraneural means.

The present invention offers a strategy to find a trade-off between high selectivity and fascicles-resolution, typically offered by intraneural interfaces (such as "Utah array"), and easy implantation and mechanical stability, typically offered by extraneural interfaces. The autonomous insertion process allows achieving intrafascicular interaction in small nerves reducing the surgical manipulation of the nerve. The morphing strategy allows inserting of high-aspect-ratio and miniaturized structures within the nerve preserving the tissue safety from damages in the long-term implant. The high-aspect-ratio feature allows significantly reducing the amount of nerve damage.

The sacrificial layer 11 allows the penetration of ultra-miniaturized and high-aspect-ratio intraneural means 2 within the nerve avoiding their bending or fracture. This results in a high fascicular resolution selectivity (spatial selectivity within the nerve cross-section) avoiding neural tissue damage and preserving nerve safety.

The 3D distribution of the intraneural means 2 both within the nerve cross-section and nerve axis allows to achieve selectivity and at the same time open the possibility to customize the stimulation and recording configuration (monopolar, bipolar, or tripolar) in a longitudinal or transversal manner.

The 3D distribution of the intraneural means 2 avoids unidirectional transversal insertion of penetrating structures within the nerve, reducing nerve fascicles trauma.

The 3D geometry of the device enables to increase the mechanical stability of the implanted interface within the nerve, avoiding possible dislocation, compared to monodimensional brittle devices.

The modularity of the geometry of the external layer, the internal layer and of the anchoring means allows the easy customization of the characteristic dimensions of the electrode to the nerve.

The modularity of the geometry enables the use of multiple fabrication techniques. Long time of lithographic process used for the greater part of neural interfaces can be avoided.

The 3D distribution of the intraneural means 2 enables to change the relative position of active sites within the transversal sections along the nerve axis by applying a rotation along the nerve axis and thus contacting more nerve fascicles and increasing the neural fascicular (spatial) selectivity.

The 3D distribution of the intraneural means 2 enables to configure the interface within the most convenient stimulation and/or recording modality (e.g.: monopolar, bipolar or tripolar configuration).

The advantages of having a "surgeon-free" implant procedure are to guarantee the repeatability of the process and reduce the risk and error margin during the procedure.

Thanks to the three-dimensionally anchoring to the nerve, the device according to the invention is less affected by the breaking of the electrode due, for example, to an undesired displacement of the device itself.

The present invention has been described with reference to preferred embodiments. However, it will be understood that variations and/or modifications can be brought to the grasper device according to the present invention without thereby departing from the scope of the invention as defined in the following claims.

### Bibliography

[1] C. E. Larson and E. Meng, "A review for the peripheral nerve interface designer," Journal of Neuroscience Methods, vol. 332, p. 108523, Feb. 2020, doi: 10.1016/j.jneumeth.2019.108523.
[2] D. A. Janssen, F. M. Martens, L. L. de Wall, H. M. van Breda, and J. P. Heesakkers, "Clinical utility of neurostimulation devices in the treatment of overactive bladder: current perspectives," Med Devices (Auckl), vol. 10, pp. 109-122, 2017, doi: 10.2147/MDER.S115678.
[3] D. J. Tyler and D. M. Durand, "A slowly penetrating interfascicular nerve electrode for selective activation of peripheral nerves," IEEE Trans. Rehab. Eng., vol. 5, no. 1, pp. 51-61, Mar. 1997, doi: 10.1109/86.559349.
[4] A. Kundu, K. R. Harreby, K. Yoshida, T. Boretius, T. Stieglitz, and W. Jensen, "Stimulation selectivity of the 'thin-film longitudinal intrafascicular electrode' (tfLIFE) and the 'transverse intrafascicular multi-channel electrode' (TIME) in the large nerve animal model," IEEE Trans Neural Syst Rehabil Eng, vol. 22, no. 2, pp. 400-410, Mar. 2014, doi: 10.1109/TNSRE.2013.2267936.
[5] R. Jung, J. Abbas, S. Kuntaegowdanahalli, and A. Thota, "Bionic intrafascicular interfaces for recording and stimulating peripheral nerve fibers," Bioelectronics in Medicine, vol. 1, pp. 55-69, Jan. 2018, doi: 10.2217/bem-2017-0009.
[6] F. M. Petrini et al., "Six-Month Assessment of a Hand Prosthesis with Intraneural Tactile Feedback: Hand Prosthesis," Annals of Neurology, vol. 85, no. 1, pp. 137-154, Jan. 2019, doi: 10.1002/ana.25384.
[7] S. Wurth et al., "Long-term usability and bio-integration of polyimide-based intra-neural stimulating electrodes," Biomaterials, vol. 122, pp. 114-129, Apr. 2017, doi: 10.1016/j.biomaterials.2017.01.014.
[8] T. S. Davis et al., "Restoring motor control and sensory feedback in people with upper extremity amputations using arrays of 96 microelectrodes implanted in the median and ulnar nerves," Journal of Neural Engineering, vol. 13, no. 3, p. 036001, Jun. 2016, doi: 10.1088/1741-2560/13/3/036001.
[9] M. B. Christensen, S. M. Pearce, N. M. Ledbetter, D. J. Warren, G. A. Clark, and P. A. Tresco, "The foreign body response to the Utah Slant Electrode Array in the cat sciatic nerve," Acta Biomater, vol. 10, no. 11, pp. 4650-4660, Nov. 2014, doi: 10.1016/j.actbio.2014.07.010.
[10] K. M. Musick et al., "Chronic multichannel neural recordings from soft regenerative microchannel electrodes during gait," Sci Rep, vol. 5, no. 1, Art. no. 1, Sep. 2015, doi: 10.1038/srep14363.
[11] "The Tissue-Engineered Electronic Nerve Interface (TEENI)," NIMET: Nanoscience Institute for Medical & Engineering Technology. https://www.eng.ufl.edu/nimet/research/projects/the-tissue-engineered-electronic-nerve-interface-teeni/ (accessed Mar. 08, 2022).
[12] B. S. Spearman et al., "Tissue-Engineered Peripheral Nerve Interfaces," Advanced Functional Materials, vol. 28, no. 12, p. 1701713, 2018, doi: 10.1002/adfm.201701713.
[13] F. Barz, P. Ruther, S. Takeuchi, and O. Paul, "Flexible silicon-polymer neural probe rigidified by dissolvable insertion vehicle for high-resolution neural recording with improved duration," in 2015 28th IEEE International Conference on Micro ElectroMechanical Systems (MEMS), Jan. 2015, pp. 636-639. doi: 10.1109/MEMSYS.2015.7051036.
[14] L. Gao et al., "Free-Standing Nanofilm Electrode Arrays for Long-Term Stable Neural Interfacings," Advanced Materials, vol. 34, no. 5, p. 2107343, 2022, doi: 10.1002/adma.202107343.
[15] A. Lecomte et al., "Silk and PEG as means to stiffen a parylene probe for insertion in the brain: toward a double time-scale tool for local drug delivery," J. Micromech. Microeng., vol. 25, no. 12, p. 125003, Dec. 2015, doi: 10.1088/0960-1317/25/12/125003.
[16] A. B. Calia et al., "Full bandwidth electrophysiology of seizures and epileptiform activity enabled by flexible graphene micro-transistor depth neural probes." bioRxiv, p. 2021.09.17.460765, Sep. 20, 2021. doi: 10.1101/2021.09.17.460765.
[17] E. Ravagli et al., "Fascicle localisation within peripheral nerves through evoked activity recordings: A comparison between electrical impedance tomography and multi-electrode arrays," Journal of Neuroscience Methods, vol. 358, p. 109140, Jul. 2021, doi: 10.1016/j.jneumeth.2021.109140.
[18] "Carbon fiber on polyimide ultra-microelectrodes - IOPscience." https://iopscience.iop.org/article/10.1088/1741-2552/aa8c88 (accessed Mar. 08, 2022).
[19] A. A. Jiman et al., "Multi-channel intraneural vagus nerve recordings with a novel high-density carbon fiber microelectrode array," Sci Rep, vol. 10, no. 1, Art. no. 1, Sep. 2020, doi: 10.1038/s41598-020-72512-7.
[20] E. J. Welle et al., "Sharpened and mechanically durable carbon fiber electrode arrays for neural interfacing." bioRxiv, p. 2021.01.21.427697, Mar. 15, 2021. doi: 10.1101/2021.01.21.427697.
[21] T. M. Otchy et al., "Printable microscale interfaces for long-term peripheral nerve mapping and precision control," Nat Commun, vol. 11, no. 1, Art. no. 1, Aug. 2020, doi: 10.1038/s41467-020-18032-4.

## Claims

1. An implantable peripheral neural interface comprising a matrix (1) adapted to be wrapped around a nerve (N) and intraneural means (2) embedded into said matrix, said matrix comprising two overlapped layers, an external layer (10) defining an outer surface (100) of the neural interface and an internal layer (11) defining an inner surface (111) of the interface, said intraneural means defining at least a penetration tip (20) adapted to penetrate at least the epinerium of said nerve the penetration tip (20) being embedded into said internal layer, said internal layer (11) being made of a biodegradable material adapted to degrade in contact with the nerve surface, the degradation of said internal layer makes exposed said penetration tip (20) of said intraneural means.

2. The implantable peripheral neural interface according to claim 1, wherein said intraneural means (2) comprise electrically conductive means.

3. The implantable peripheral neural interface according to claim 1 or 2, wherein said intraneural means (2) comprise a penetration portion (200) comprising said penetration tip (20) and a non-penetration portion (210) that remains out of the nerve during implantation, said penetration portion (200) being embedded into said internal layer (10), said non-penetration portion (210) being associated to said external layer (11).

4. The implantable peripheral neural interface according to claim 3 wherein said intraneural means (2) comprise a fixed tip (21) opposite to said penetration tip (20), said fixed tip (21) being stable connected to said matrix.

5. The implantable peripheral neural interface according to claim 4, wherein said fixed tip (21) is connected to an interfacial surface (110) between said internal and external layer.

6. The implantable peripheral neural interface according to claim 4 wherein said fixed tip (21) is embedded into said external layer.

7. The implantable peripheral neural interface according to claim 4 wherein said fixed tip (21) is connected to said outer surface (100).

8. The implantable peripheral neural interface according to any of the previous claims wherein said external layer provides one or more layers.

9. The implantable peripheral neural interface according to any of the previous claims wherein said external layer (10) is made of a biocompatible material with elastic and insulating properties.

10. The implantable peripheral neural interface according to claim 9 wherein said material is polydimethylsiloxane (PDMS).

11. The implantable peripheral neural interface according to any of the previous claims wherein said degradable material of said internal layer is a biocompatible biodegradable polymer.

12. The implantable peripheral neural interface according to claim 11 wherein said internal layer is made of polyglycerol sebacate.

13. The implantable peripheral neural interface according to any of the previous claims wherein said intraneural means comprise one or more micrometric high-aspect-ratio conductive linear elements.

14. The implantable peripheral neural interface according to any of the previous claims wherein suturing means (12) for the stable fixing of the electrode to the nerve are associated to the external layer (10).

15. The implantable peripheral neural interface according to claim 14 wherein suturing means (12) comprises suturing wires projecting by the opposite end of the external layer.

## Patentansprüche

1. Implantierbare periphere neuronale Schnittstelle, umfassend eine Matrix (1), die angepasst ist, um einen Nerv (N) zu umhüllen, und intraneuronale Mittel (2), die in die Matrix eingebettet sind, die Matrix umfassend zwei überlagerte Schichten, eine externe Schicht (10), die eine äußere Oberfläche (100) der neuronalen Schnittstelle definiert, und eine interne Schicht (11), die eine innere Oberfläche (111) der Schnittstelle definiert, wobei die intraneuronalen Mittel mindestens eine Penetrationsspitze (20) definieren, die angepasst ist, um mindestens das Epineurium des Nervs zu penetrieren, wobei die Penetrationsspitze (20) in die interne Schicht eingebettet ist, wobei die interne Schicht (11) aus einem biologisch abbaubaren Material hergestellt ist, das angepasst ist, um sich bei Kontakt mit der Nervenoberfläche abzubauen, wobei der Abbau der internen Schicht die Penetrationsspitze (20) der intraneuronalen Mittel freilegt.

2. Implantierbare periphere neuronale Schnittstelle nach Anspruch 1, wobei die intraneuronalen Mittel (2) elektrisch leitfähige Mittel umfassen.

3. Implantierbare periphere neuronale Schnittstelle nach Anspruch 1 oder 2, wobei die intraneuronalen Mittel (2) einen Penetrationsabschnitt (200), umfassend die Penetrationsspitze (20), und einen Nicht-Penetrationsabschnitt (210), der während einer Implantation außerhalb des Nervs verbleibt, umfassen, wobei der Penetrationsabschnitt (200) in die interne Schicht (10) eingebettet ist und der Nicht-Penetrationsabschnitt (210) der externen Schicht (11) zugehörig ist.

4. Implantierbare periphere neuronale Schnittstelle nach Anspruch 3, wobei die intraneuronalen Mittel (2) eine der Penetrationsspitze (20) entgegengesetzte fixierte Spitze (21) umfassen, wobei die fixierte Spitze (21) mit der Matrix stabil verbunden ist.

5. Implantierbare periphere neuronale Schnittstelle nach Anspruch 4, wobei die fixierte Spitze (21) mit einer Grenzflächenoberfläche (110) zwischen der internen und der externen Schicht verbunden ist.

6. Implantierbare periphere neuronale Schnittstelle nach Anspruch 4, wobei die fixierte Spitze (21) in die externe Schicht eingebettet ist.

7. Implantierbare periphere neuronale Schnittstelle nach Anspruch 4, wobei die fixierte Spitze (21) mit der äußeren Oberfläche (100) verbunden ist.

8. Implantierbare periphere neuronale Schnittstelle nach einem der vorstehenden Ansprüche, wobei die externe Schicht eine oder mehrere Schichten bereitstellt.

9. Implantierbare periphere neuronale Schnittstelle nach einem der vorstehenden Ansprüche, wobei die externe Schicht (10) aus einem biokompatiblen Material mit elastischen und isolierenden Eigenschaften hergestellt ist.

10. Implantierbare periphere neuronale Schnittstelle nach Anspruch 9, wobei das Material Polydimethylsiloxan (PDMS) ist.

11. Implantierbare periphere neuronale Schnittstelle nach einem der vorstehenden Ansprüche, wobei das abbaubare Material der internen Schicht ein biokompatibles biologisch abbaubares Polymer ist.

12. Implantierbare periphere neuronale Schnittstelle nach Anspruch 11, wobei die interne Schicht aus Polyglycerolsebacat hergestellt ist.

13. Implantierbare periphere neuronale Schnittstelle nach einem der vorstehenden Ansprüche, wobei die intraneuronalen Mittel ein oder mehrere mikrometrische leitfähige lineare Elemente mit hohem Seitenverhältnis umfassen.

14. Implantierbare periphere neuronale Schnittstelle nach einem der vorstehenden Ansprüche, wobei Nahtmittel (12) zum stabilen Fixieren der Elektrode an dem Nerv der externen Schicht (10) zugehörig sind.

15. Implantierbare periphere neuronale Schnittstelle nach Anspruch 14, wobei die Nahtmittel (12) Nahtdrähte umfassen, die an dem entgegengesetzten Ende der externen Schicht hervorstehen.

## Revendications

1. Interface neurale périphérique implantable comprenant une matrice (1) conçue pour être enveloppée autour d'un nerf (N) et des moyens intraneuraux (2) intégrés dans ladite matrice, ladite matrice comprenant deux couches en chevauchement, une couche externe (10) définissant une surface extérieure (100) de l'interface neurale et une couche interne (11) définissant une surface interne (111) de l'interface, lesdits moyens intraneuraux définissant au moins une pointe de pénétration (20) conçue pour pénétrer au moins dans l'épinèvre dudit nerf la pointe de pénétration (20) étant intégrée dans ladite couche interne, ladite couche interne (11) étant fabriquée dans un matériau biodégradable conçu pour se dégrader en contact avec la surface nerveuse, la dégradation de ladite couche interne rend exposée ladite pointe de pénétration (20) desdits moyens intraneuraux.

2. Interface neurale périphérique implantable selon la revendication 1, dans laquelle lesdits moyens intraneuraux (2) comprennent des moyens électriquement conducteurs.

3. Interface neurale périphérique implantable selon la revendication 1 ou 2, dans laquelle lesdits moyens intraneuraux (2) comprennent une partie de pénétration (200) comprenant ladite pointe de pénétration (20) et une partie non pénétrante (210) qui reste à l'extérieur du nerf pendant l'implantation, ladite partie de pénétration (200) étant intégrée dans ladite couche interne (10), ladite partie non pénétrante (210) étant associée à ladite couche externe (11).

4. Interface neurale périphérique implantable selon la revendication 3 dans laquelle lesdits moyens intraneuraux (2) comprennent une pointe fixe (21) opposée à ladite pointe de pénétration (20), ladite pointe fixe (21) étant reliée de façon stable à ladite matrice.

5. Interface neurale périphérique implantable selon la revendication 4, dans laquelle ladite pointe fixe (21) est reliée à une surface interfaciale (110) entre lesdites couches interne et externe.

6. Interface neurale périphérique implantable selon la revendication 4 dans laquelle ladite pointe fixe (21) est intégrée dans ladite couche externe.

7. Interface neurale périphérique implantable selon la revendication 4 dans laquelle ladite pointe fixe (21) est reliée à ladite surface extérieure (100).

8. Interface neurale périphérique implantable selon l'une quelconque des revendications précédentes dans laquelle ladite couche externe fournit une ou plusieurs couches.

9. Interface neurale périphérique implantable selon l'une quelconque des revendications précédentes dans laquelle ladite couche externe (10) est fabriquée dans un matériau biocompatible avec des propriétés élastiques et isolantes.

10. Interface neurale périphérique implantable selon la revendication 9 dans laquelle ledit matériau est du polydiméthylsiloxane (PDMS).

11. Interface neurale périphérique implantable selon l'une quelconque des revendications précédentes dans laquelle ledit matériau dégradable de ladite couche interne est un polymère biodégradable biocompatible.

12. Interface neurale périphérique implantable selon la revendication 11 dans laquelle ladite couche interne est fabriquée en sébaçate de polyglycérol.

13. Interface neurale périphérique implantable selon l'une quelconque des revendications précédentes dans laquelle lesdits moyens intraneuraux comprennent un ou plusieurs éléments linéaires micrométriques à rapport d'aspect élevé.

14. Interface neurale périphérique implantable selon l'une quelconque des revendications précédentes dans laquelle des moyens de suture (12) pour la fixation stable de l'électrode au nerf sont associés à la couche externe (10).

15. Interface neurale périphérique implantable selon la revendication 14 dans laquelle les moyens de suture (12) comprennent des fils de suture faisant saillie par l'extrémité opposée de la couche externe.
